(19) Europäisches Patentamt

European Patent Office

Office européen des brevets

(11) Numéro de publication: **0 217 695**
**B1**

(12) **FASCICULE DE BREVET EUROPEEN**

(45) Date de publication du fascicule du brevet:
08.02.89

(51) Int. Cl.⁴: **C 07 C 6/12, B 01 J 29/18**

(21) Numéro de dépôt: 86401850.2

(22) Date de dépôt: 21.08.86

(54) Procédé de dismutation et transalkylation d'hydrocarbures aromatiques en présence d'un catalyseur zéolithique.

(30) Priorité: 28.08.85 FR 8512916

(43) Date de publication de la demande:
08.04.87 Bulletin 87/15

(45) Mention de la délivrance du brevet:
08.02.89 Bulletin 89/6

(84) Etats contractants désignés:
DE GB IT NL

(56) Documents cités:
FR-A- 2 159 320
FR-A- 2 377 226
FR-A- 2 471 359
US-A- 4 278 827

(73) Titulaire: INSTITUT FRANCAIS DU PETROLE, 4, Avenue de Bois-Préau, F-92502 Rueil-Malmaison (FR)

(72) Inventeur: Dufresne, Pierre, 67, rue Georges Sand, F-92500 Rueil Malmaison (FR)
Inventeur: Marcilly, Christian, 91 ter rue Condorcet, F-78800 Houilles (FR)
Inventeur: Raatz, Francis, 17, rue Michelet, F-92500 Rueil Malmaison (FR)

ACTORUM AG

## Description

La présente invention a trait à la transformation catalytique des hydrocarbures aromatiques. D'une manière plus précise, elle concerne la dismutation d'hydrocarbures alkylaromatiques tels que le toluène, pour produire du benzène et des xylènes, ou la transalkylation d'hydrocarbures alkylaromatiques tels que le toluène et les triméthylbenzènes pour produire des xylènes. Ces réactions sont effectuées en présence d'un catalyseur et généralement dans les conditions opératoires suivantes: température comprise entre 250 et 550° C et de préférence entre 330 et 500° C; pression comprise entre 10 et 60 bar et de préférence entre 20 et 45 bar; vitesse spatiale d'alimentation, exprimée en grammes de charge introduite par gramme de catalyseur et par heure, comprise entre 0,1 et 10 et de préférence entre 0,5 et 4; rapport molaire hydrogène sur hydrocarbures compris entre 2 et 20 et de préférence entre 3 et 12.

L'objet de la présente invention concerne l'utilisation d'un catalyseur particulier qui se révèle très efficace pour la dismutation du toluène et/ou la transalkylation du toluène et des hydrocarbures alkylaromatiques en $C_9^+$ (c'est-à-dire à 9, ou davantage, atomes de carbone par molécule).

Le catalyseur utilisé se révèle hautement sélectif et présente une activité et une stabilité améliorées par rapport aux catalyseurs de l'art antérieur. Ce catalyseur, prêt à l'emploi, est constitué d'une mordénite appartenant à la catégorie «larges pores», c'est-à-dire dont le diamètre des pores principaux est d'environ $7 \times 10{-}10$ m à environ $10 \times 10{-}10$ m (7 à 10 angströms) ayant un rapport molaire $SiO_2/Al_2O_3$ compris entre 9 et 80 et de préférence entre 12 et 60 et très avantageusement entre 20 et 50, contenant moins de 1% poids de sodium (en poids) et de préférence moins de 0,5% de sodium, et comprenant également d'une part un ou plusieurs métaux de transition choisis dans le groupe constitué par les métaux des groupes IB et VIII de la classification périodique des éléments (Handbook of Chemistry and Physics, 37e édition 1955-56, pages 392-393) et de préférence choisis parmi le nickel, l'argent et le palladium, et d'autre part un ou plusieurs métaux du groupe IVA, notamment germanium, étain et plomb. De nombreux catalyseurs de dismutation et transalkylation à base de mordénite ont déjà été décrits dans l'art antérieur. La mordénite utilisée est en général une mordénite dite «larges pores», c'est-à-dire dont le diamètre de pores est de l'ordre de $7 \times 10{-}10$ m (7 angströms), ce diamètre étant de l'ordre de $8 \times 10{-}10$ m à $10 \times 10{-}10$ m (8 à 10 angströms) dans le catalyseur prêt à l'emploi: il en est ainsi dans le brevet des Etats Unis d'Amérique N° 3 506 731 où l'on utilise une mordénite sous sa forme hydrogène ou dans les deux demandes de brevets français du demandeur N° 2 329 619 et N° 2 367 533. Il en est encore ainsi dans le brevet des Etats-Unis d'Amérique N° 3 281 483 qui fait mention de mordénites échangées essentiellement avec des ions argent ou nickel ou dans le brevet US N° 3 780 121 qui fait état d'une mordénite échangée avec des ions des métaux du groupe IB de la classification périodique des éléments (cuivre, argent) et est caractérisée par un rapport molaire $SiO_2/Al_2O_3$ compris entre 12 et 80 ou encore dans le brevet US N° 3 629 351 qui concerne également une mordénite contenant des ions des métaux des groupes IB, VA, VIA, IIA et VIII de la classification périodique des éléments, ou enfin dans le brevet US N° 3 476 821 qui décrit l'utilisation d'une mordénite contenant un métal du groupe VIII sulfuré et ayant de préférence un rapport molaire $SiO_2/Al_2O_3$ supérieur à 20. Selon l'invention, la zéolithe utilisée comme base pour préparer le catalyseur est une mordénite sodique appartenant à la catégorie des mordénites dites «à larges pores», c'est-à-dire des mordénites dont le diamètre des pores principaux est d'environ $7 \times 10{-}10$ m à environ $10 \times 10{-}10$ m (7 à 10 angströms), ou à petits pores, c'est-à-dire des mordénites dont le diamètre des pores principaux est d'environ $4 \times 10{-}10$ m à environ $6 \times 10{-}10$ m (4 à 6 angströms) sous la forme sodique. Pour la fabrication des catalyseurs décrits dans les exemples, on a utilisé une mordénite en poudre ou extrudée sous forme sodique, commercialisée sous le nom de zéolon par la société américaine NORTON, de structure orthorhombique, de surface spécifique 400 à 500 m² · g−1 environ, pouvant adsorber jusqu'à 13,5% en poids d'eau et dont le diamètre moyen de pores est $7 \times 10{-}10$ m (7 angströms) environ. Cette ouverture des pores permet ainsi à cette mordénite d'absorber, en deux heures, à 28° C sous 3333,05 Pa (25 mm de mercure), dans sa microporosité de structure, entre 8 et 9% en poids environ d'hydrocarbures aromatiques simples tels que du benzène ou du toluène dont l'encombrement stérique est de $6,8 \times 10{-}10$ m (6,8 angströms), entre 7 et 8% environ en poids d'orthoxylène (en deux heures, à 28° C sous 933,26 Pa (7 mm de mercure)) ou 6 à 7% environ en poids de 2,2,4 triméthylpentane (isooctane) sous 4933 Pa (37 mm de mercure), à 28° C. La présente invention est un procédé amélioré de dismutation ou de transalkylation d'hydrocarbures alkylaromatiques dans lequel on opère en présence d'un catalyseur renfermant:

a) au moins une mordénite acide dont le rapport molaire global $SiO_2/Al_2O_3$ est de 9 à 80 et de préférence de 12 à 60 et très avantageusement de 20 à 50 et dont la teneur en sodium est inférieure à 1% en poids et de préférence inférieure à 0,5% en poids, et très avantageusement inférieure à environ 0,25% en poids,

b) au moins un métal choisi dans le groupe formé par le nickel, le palladium et les métaux du groupe IB de la classification périodique des éléments et de préférence parmi le nickel, le palladium et l'argent,

c) au moins un métal choisi parmi les métaux du groupe IVA de la classification périodique des éléments et de préférence parmi l'étain, le plomb et le germanium. Le catalyseur employé dans la présente invention contient de préférence de 30 à 99,9% en poids de mordénite et très avantageusement de 50 à 99,5%. Pour préparer le catalyseur utilisé dans la présente invention, il convient tout d'abord d'extraire la majeure partie des ions de sodium de la mordénite; de préférence, la mordénite ne doit pas renfermer plus de 1% en poids d'ions sodium et de préférence pas plus de 0,5% en poids d'ions sodium. Une première méthode consiste à remplacer ces ions sodium tout d'abord par des ions ammonium par exemple entre 0 et 120°C, à l'aide d'une ou de plusieurs réactions d'échange ionique successives. Une deuxième méthode consiste à soumettre le solide à un ou plusieurs traitements successifs dans une solution d'un acide minéral tel que HCl, $H_2SO_4$ ou $HNO_3$ par exemple, de concentration comprise par exemple entre 0,3 et 8 M pendant 30 minutes à 24 heures entre 20 et 120°C. Avec cette dernière méthode, simultanément à l'échange ionique, c'est-à-dire au remplacement du sodium par des protons, se produit une réaction d'extraction d'aluminium de la charpente alumino-silicatée qui se traduit par une augmentation du rapport molaire $SiO_2/Al_2O_3$ de cette dernière. Une troisième méthode consiste à utiliser une combinaison des deux précédentes; par exemple, on effectue une ou plusieurs opérations d'échange ionique à l'aide d'une solution d'un sel d'ammonium puis on fait subir au solide un ou plusieurs traitements en milieu acide tels qu'ils ont été décrits précédemment. Il est également possible d'opérer le ou les traitements en milieu acide avant le ou les échanges ioniques. Après élimination de la majeure partie du sodium par l'une des techniques précédemment décrites et dans le but d'améliorer ses propriétés acides et donc ses performances, le support zéolithique utilisé dans la présente invention subit ensuite une calcination en présence de vapeur d'eau (calcination dite humide) à une température comprise entre 300 et 850°C, de préférence entre 400 et 800°C, ou mieux entre 450 et 750°C, pendant une durée variant de 15 minutes à une semaine, de préférence de 30 minutes à 10 heures. La pression partielle de vapeur d'eau est comprise entre 0,02 et 5 atmosphères (0,002-0,50 mégapascals (MPa)). Ce traitement se traduit comme précédemment par une extraction d'aluminium de la charpente alumino-silicatée et donc par une augmentation du rapport molaire $SiO_2/Al_2O_3$ de cette dernière mais, contrairement aux traitements en milieu acide qui permettent de retirer l'aluminium ainsi extrait hors de la structure, les calcinations humides laissent l'aluminium extrait dans les canaux de la zéolithe, si bien que le rapport $SiO_2/Al_2O_3$ global du solide ne change pas de manière sensible. Les calcinations de mordénite désodée en présence de vapeur d'eau sont connues depuis longtemps et ont été décrites notamment dans les brevets US 3 506 400 et US 3 551 353 et dans la publication de CHEN et SMITH, Inorg. Chem., 1976, 15, p. 295.

Après cette première calcination, il est possible et même préférable de procéder à une attaque en milieu acide du même type que celles décrites précédemment, de manière à retirer la majeure partie de l'aluminium extrait de la charpente alumino-silicatée mais encore présent dans les canaux de la zéolithe.

Chacun des traitements décrits ici, qu'il s'agisse de la calcination sous air humide ou de l'attaque acide, se traduit par un enrichissement en silice de la charpente alumino-silicatée, mais, comme nous l'avons dit, seule l'attaque acide permet d'augmenter le rapport $SiO_2/Al_2O_3$ global de la zéolithe. Les valeurs désirées pour la présente invention du rapport molaire global $SiO_2/Al_2O_3$ de la zéolithe peuvent ainsi être obtenues en procédant à un ou plusieurs cycles successifs calcination air humide-attaque acide. Lorsqu'une seule calcination sous air humide est réalisée, elle est de préférence suivie d'une attaque acide de façon à atteindre un rapport molaire global $SiO_2/Al_2O_3$ de préférence d'au moins 12. En revanche, si plusieurs calcinations sous air humide sont réalisées, le dernier traitement auquel sera soumise la zéolithe peut être indifféremment soit la calcination, soit le traitement en milieu acide. L'utilisation de cycles de traitements alternés calcination air humide - attaque acide a également été décrite dans les brevets US 3 551 353, GB 1 061 847 et dans les publications de CHEN et SMITH, Inorg. Chem., 1976, 15, p. 295; de CHEN, J. Phys. Chem., 1976, 80, p. 60 et OLSON et ROLLMANN, Inorg. Chem., 1977, 16, p. 651.

On procède ensuite à l'introduction de l'un au moins des éléments de transition appartenant soit au groupe IB de la classification périodique, notamment l'argent, soit au groupe formé par le nickel et la palladium. On peut introduire le ou les métaux désirés (argent, nickel ou palladium) soit par imprégnation du solide sec, soit de manière préférée par échange ionique. Si le catalyseur contient deux ou trois des métaux argent, nickel ou palladium, ces métaux peuvent être introduits soit tous de la même façon, soit par des méthodes identiques pour deux d'entre eux et différentes pour le troisième. Dans le cas où la technique d'échange ionique est adoptée, les ions suivants des métaux seront de préférence utilisés: $Ag^+$ ou $Ag(NH_3)_2^+$, $Ni^{2+}$ ou $Ni(NH_3)_n^{2+}$ où $n=2$, 4 ou 6, $Pd^{2+}$ ou $Pd(NH_3)_4^{2+}$.

Les acétates de ces métaux peuvent également être avantageusement employés pour l'opération d'échange lorsque cette dernière est réalisée sur une mordénite forme acide, c'est-à-dire une mordénite dont la plupart des cations de compensation sont des ions $H^+$. Plusieurs échanges successifs peuvent être nécessaires pour introduire les quantités requises en métaux.

Après introduction des éléments métalliques du groupe IB de la classification périodique et/ou du groupe formé par le nickel et le palladium, le solide obtenu est lavé, filtré et séché pendant une durée pouvant varier indifféremment de quelques minutes à quelques jours, entre 80 et 200°C selon la durée adoptée pour le séchage, les séchages les plus longs étant réalisés de préférence aux températures les plus basses. Le produit est ensuite calciné sous air normal ou sous air séché pendant 30 minutes à 20 heures et de préférence 1 à 10 heures entre 400 et 700°C et de préférence entre 450 et 600°C.

On peut alors procéder à l'introduction des éléments métalliques du groupe IVA de la classification périodique. Cette opération est réalisée de préférence à l'aide de complexes organiques dans lesquels les métaux sont présents de préférence à un degré d'oxydation de 0 ou 2 par exemple. Parmi les 3 métaux du groupe IVA qui conviennent pour la présente invention, germanium, étain et plomb, les deux métaux préférés sont l'étain et le plomb. Parmi les nombreux complexes possibles pour introduire ces métaux, on emploie de préférence les alkyls métaux et notamment les alkyls étain et les alkyls plomb. On peut citer par exemple pour l'étain: le tétraméthylétain, le tétraéthylétain, les tétrapropylétains, les tétrabutylétains, le dichlorure d'étain diméthyl et le dichlorure d'étain dibutyl et, pour le plomb, le tétraéthyl plomb qui est un produit de grande consommation.

Les complexes organiques possibles sont mis en solution aqueuse ou organique, placés en atmosphère neutre ou réductrice, au contact de la mordénite issue des traitements décrits précédemment et l'ensemble est porté pendant plusieurs minutes à plusieurs jours, de préférence entre 5 minutes et 72 heures, à une température comprise entre 0 et 150° C en phase liquide jusqu'à décomposition de la quantité désirée du complexe. Dans la quasi-totalité des cas, la décomposition du complexe organique est accélérée par la présence des éléments métalliques déposés précédemment, notamment argent, nickel ou palladium. On a cependant constaté dans la présente invention que la vitesse de décomposition de ces complexes sur les mordénites échangées calcinées sous air sont faibles, notamment dans le cas de la mordénite échangée au nickel, et qu'il peut être préférable de réduire préalablement le solide en présence d'un gaz réducteur, notamment l'hydrogène ou un gaz riche en hydrogène, entre 300 et 700° C, de préférence entre 400 et 600° C pendant environ 30 minutes à 48 heures. Une telle réduction accroît de manière très sensible la vitesse de décomposition du complexe organique du métal du groupe IVA et donc la vitesse de fixation de l'élément sur le support.

Les teneurs totales en ions des métaux de transition des groupes IB (Ag notamment) ou Ni, Pd dans les mordénites peuvent être de 0,005 à 25% poids, les teneurs préférées étant les suivantes: 0,1 à 21% poids pour l'argent, 0,1 à 3,5% poids pour le nickel et 0,005 à 2% poids pour le palladium.

Les teneurs totales en ions des métaux du groupe IVA (Ge, et notamment Sn et Pb) dans les mordénites acides peuvent être de 0,05 à 10% en poids, les teneurs préférées étant de 0,1 à 4% en poids.

Dans la présente invention, le catalyseur est de préférence utilisé sous forme de pastilles, d'agrégats, d'extrudés ou de billes. La mise en forme du catalyseur peut être réalisée en l'absence de liant. Elle peut être aussi réalisée avant ou après les opérations d'échange en utilisant un liant qui peut être choisi parmi les liants classiques tels que les argiles, les alumines, la silice et les silice-alumines. Dans ce dernier cas, les liants préférés dans la présente invention sont certaines argiles et/ou alumines, utilisés par exemple à raison de 3 à 50% poids et, de manière préférée, de 5 à 40% poids.

Le catalyseur préparé selon la présente invention présente des propriétés catalytiques nettement améliorées par rapport aux catalyseurs traditionnels dans les opérations de dismutation du toluène en benzène et xylènes et de transalkylation du toluène par les triméthylbenzènes. Les catalyseurs utilisés de manière classique pour ces opérations qui sont des mordénites acides échangées avec des métaux du groupe IB, notamment l'argent, ou du groupe VIII, notamment le nickel, présentent deux inconvénients majeurs: une sélectivité insuffisante qui se traduit par une légère hydrogénation et dégradation des composés aromatiques en produits saturés plus légers et une stabilité dans le temps insuffisante encore difficilement acceptable sur le plan industriel. Les catalyseurs de la présente invention apportent des améliorations très substantielles sur ces deux plans: la perte en noyaux benzéniques à chaque passage dans le réacteur est ainsi divisée par un facteur allant de environ 2 à environ 20 et la stabilité dans le temps du catalyseur, mesurée par exemple par la durée en jours correspondant à une augmentation de 30° C pour maintenir la conversion du toluène à 40%, est multipliée par un facteur d'environ 1,5 à 4.

Le catalyseur utilisé dans la présente invention, lorsqu'il est retiré de l'unité de test après un test de plusieurs semaines, contient entre environ 0,5 et 4% en poids de coke. Ce coke peut être facilement éliminé par combustion lente sous air appauvri jusqu'à une température de 550° C. Le catalyseur ainsi régénéré retrouve en majeure partie ses propriétés catalytiques initiales.

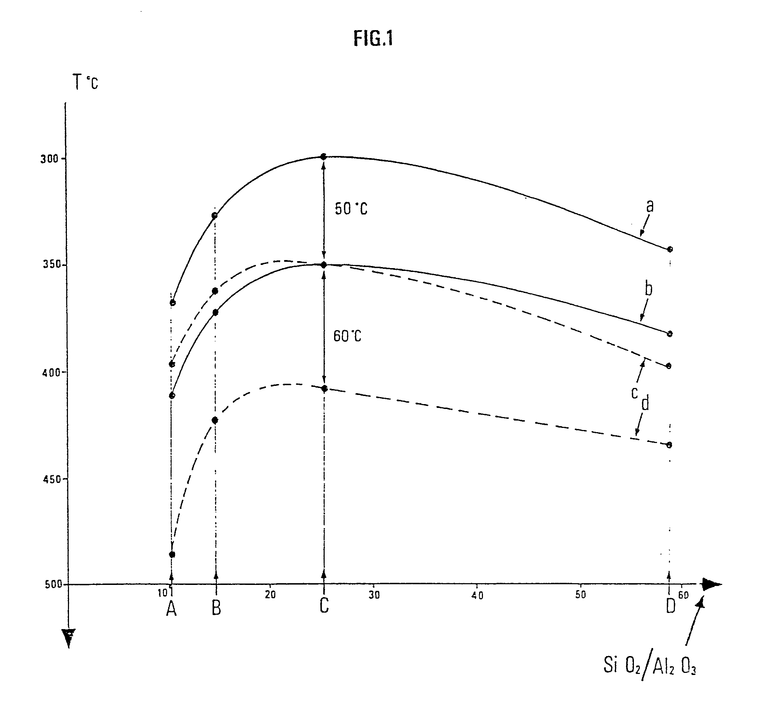

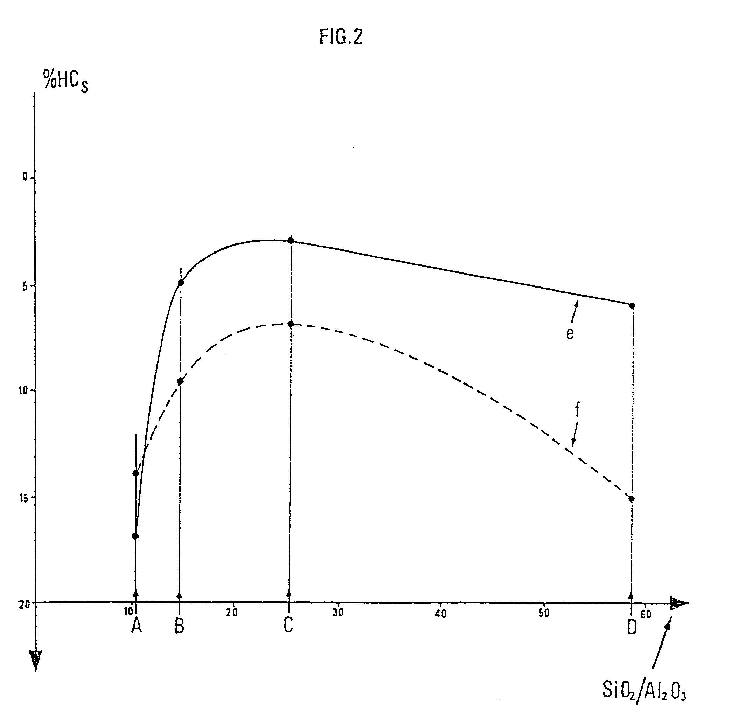

Les activités des divers catalyseurs préparés dans les exemples ci-dessous sont illustrées dans les figures 1 et 2. Sur la figure 1 on a représenté les activités et les stabilités des catalyseurs A à D en fonction de la température pour une conversion de 10% (courbes a et c) et pour une conversion de 45% (courbes b et d), avant veillissement (courbes a et b) et après vieillissement (courbes c et d).

La figure 2 représente les sélectivités des catalyseurs A à D pour une conversion de 45% avant vieillissement (courbe e) et après vieillissement (courbe f). Sur l'axe des «y» est porté le pourcentage d'hydrocarbures saturés de $C_1$ à $C_9$ obtenus (% HC$_s$).

La figure 3 représente les activités et les stabilités des catalyseurs A à D et F à I en fonction de la température pour une conversion de 10% (courbes a et g) et pour une conversion de 45% (courbes b et h) avant vieillissement et pour une conversion de 45% après vieillissement (courbes i et d).

Les courbes a, b et d sont celles des catalyseurs A à D et les courbes g, h et i sont celles des catalyseurs contenant de l'étain F à I.

La figure 4 représente les activités des catalyseurs $A_o$ à $D_o$ en fonction de la température pour une conversion de 10% (courbes j et l) et pour une conversion de 40% (courbe k). Les courbes j et k sont représentatives de l'activité des catalyseurs avant vieillissement. La courbe l représente l'activité des catalyseurs après vieillissement.

La figure 5 représente les sélectivités des catalyseurs $A_o$ à $D_o$ pour une conversion de 40% avant vieillissement des catalyseurs (courbe m).

Les exemples suivants illustrent l'invention. Les exemples 1 à 4 concernent la préparation de mordénites acides de différents rapports molaires $SiO_2/Al_2O_3$ utilisées comme catalyseurs de référence $A_o$ à $D_o$. Les exemples 5 à 9 concernent la préparation de catalyseurs de référence A à D contenant du nickel (exemples comparatifs). Les exemples 10 à 15 concernent la préparation de catalyseurs selon la présente invention. L'exemple 16 concerne la préparation de catalyseurs de référence L, N, O et P contenant de l'étain (exemple comparatif). Les exemples 17 à 23 concernent l'utilisation des catalyseurs dans la réaction de dismutation du toluène en phase vapeur.

*Exemple 1 :*

Préparation d'une mordénite acide de rapport molaire global $SiO_2/Al_2O_3 = 10,6$.

900 grammes de mordénite sodique (zéolon 900 Na) en extrudés de 1,587 mm (1/16 pouce) commercialisée par la société américaine NORTON, sont soumis à deux échanges ioniques dans une solution de nitrate d'ammonium dans les conditions suivantes :

| | |
|---|---|
| volume de solution | 3,6 litres |
| concentration de la solution en $NO_3NH_4$ | 2 M |
| durée de l'échange | 1 heure |
| température de l'échange | T=90° C. |

Après chaque échange, le solide est lavé dans 1,8 litre d'eau distillée pendant 20 minutes à 20° C.

A la fin de ces opérations, le produit est séché à 120° C dans une étuve pendant 3 heures. Le solide ainsi obtenu est calciné pendant 2 heures à 550° C sous un débit de 180 litres par heure d'un air humide contenant 50% en volume de vapeur d'eau.

La mordénite forme hydrogène ($M_1$) résultant de cette calcination est de nouveau soumise à un échange ionique dans une solution de nitrate d'ammonium dans les mêmes conditions que celles décrites précédemment. Elle est filtrée, lavée à l'eau distillée comme précédemment, séchée à 120° C puis calcinée 2 heures à 500° C sous air sec.

Les deux caractéristiques essentielles de ce produit ($M_2$) sont :
rapport molaire global $SiO_2/Al_2O_3 = 10,6$
% poids de sodium résiduel = 0,22.

Ce produit ($M_2$) est appelé, dans les tests ci-après, catalyseur $A_o$.

*Exemple 2 :*

Préparation d'une mordénite acide de rapport molaire global $SiO_2/Al_2O_3 = 14,6$.

Une nouvelle quantité de mordénite sodique zéolon 900 Na est soumise au traitement décrit dans l'exemple 1 pour préparer la mordénite forme hydrogène ($M_1$).

La mordénite forme hydrogène ($M_1$) résultant de la calcination sous air humide décrite dans l'exemple 1 est plongée dans 9 litres d'une solution 0,6M en acide chlorhydrique et est traitée pendant 2 heures à 90° C dans cette solution.

Après un lavage de 10 minutes dans 5,4 litres d'eau distillée, puis filtration, le produit est séché à 120° C pendant 2 heures, puis est partagé en 3 parties de poids identiques.

Les deux caractéristiques essentielles de ce produit ($M_3$) sont :
rapport molaire global $SiO_2/Al_2O_3 = 14,6$
% poids de sodium résiduel = 0,20

Ce produit ($M_3$) est appelé, dans les tests ci-après, catalyseur $B_o$.

*Exemple 3 :*

Préparation d'une mordénite acide de rapport molaire global $SiO_2/Al_2O_3 = 25,2$

Deux des trois parties, soit 600 grammes, de la mordénite acide ($M_3$) obtenue dans l'exemple 2 sont soumises à une calcination sous air humide dans des conditions comparables à celles de l'exemple 1, sauf en ce qui concerne la température qui est portée à 600° C.

Le solide obtenu est plongé dans 3,6 litres d'une solution aqueuse normale en acide chlorhydrique et est traité pendant 2 heures à 90° C dans cette solution.

Après un lavage de 10 minutes dans 3,6 litres d'eau distillée à 20° C, le solide est séché à 120° C pendant 2 heures.

Les deux caractéristiques essentielles de cette mordénite acide ($M_4$) sont :
rapport molaire global $SiO_2/Al_2O_3 = 25,2$
% poids de sodium résiduel = 0,11

Ce produit ($M_4$) est appelé, dans les tests ci-après, catalyseur $C_o$.

*Exemple 4 :*

Préparation d'une mordénite acide de rapport molaire global $SiO_2/Al_2O_3 = 58,6$

300 grammes de la mordénite acide ($M_4$) obtenue dans l'exemple 3 sont soumis à une calcination sous air humide dans des conditions comparables à celles de l'exemple 1, sauf en ce qui concerne la température qui est portée à 650° C.

Le solide obtenu est plongé dans 1,8 litre d'une solution aqueuse 2N en acide chlorhydrique et est traité pendant 2 heures à 90° C dans cette solution.

Le solide est ensuite lavé, filtré et séché comme dans l'exemple 2. Les deux caractéristiques essentielles de la mordénite acide ($M_5$) obtenue sont :
rapport molaire global $SiO_2/Al_2O_3 = 58,6$
% poids de sodium résiduel = 0,09

Ce produit ($M_5$) est appelé, dans les tests ci-après, catalyseur $D_o$.

*Exemple 5 :*

Préparation du catalyseur A de référence Ni-M2

100 grammes de la mordénite acide ($M_2$) de l'exemple 1 sont plongés et agités dans un volume de 900 $cm^3$ d'une solution aqueuse 0,5M d'acétate de nickel à 20° C pendant 24 heures.

Le solide obtenu est lavé à l'eau distillée, filtré puis séché à 120° C pendant une nuit. Il est ensuite calciné sous air très sec à 500° C pendant 2 heures.

Le catalyseur A ainsi obtenu a une teneur en nickel de 1,81% en poids.

*Exemple 6:*

Préparation du catalyseur B de référence Ni-M3.

100 grammes de la mordénite acide ($M_3$) de l'exemple 2 sont soumis aux mêmes traitements que ceux décrits dans l'exemple 5. Le catalyseur B ainsi obtenu a une teneur en nickel de 2,11% en poids.

*Exemple 7:*

Préparation du catalyseur C de référence Ni-M4

100 grammes de la mordénite acide ($M_4$) de l'exemple 3 sont soumis aux mêmes traitements que ceux décrits dans l'exemple 5. Le catalyseur C ainsi obtenu a une teneur en nickel de 1,10% en poids.

*Exemple 8:*

Préparation du catalyseur D de référence Ni-M5.

100 grammes de la mordénite acide ($M_5$) de l'exemple 4 sont soumis aux mêmes traitements que ceux décrits dans l'exemple 5. Le catalyseur D ainsi obtenu a une teneur en nickel de 0,43% en poids.

*Exemple 9:*

Préparation du catalyseur E de référence Pd-M4

100 grammes de la mordénite acide ($M_4$) de l'exemple 3 sont plongés dans 500 cm³ d'une solution contenant 0,15 g de palladium sous forme de chlorure de palladium tétrammine $Cl_2 Pd (NH_3)_4$, et 100 grammes de nitrate d'ammonium. Le mélange est agité pendant 24 heures à 20° C. A l'issue de ce traitement, le solide est lavé à l'eau distillée, filtré, séché à 120° C pendant une nuit, puis calciné sous air très sec à 500° C pendant 2 heures. Le catalyseur E ainsi obtenu a une teneur en palladium de 0,1% en poids.

*Exemple 10:*

Préparation du catalyseur F (Sn-Ni-M2)

50 grammes du catalyseur A (Ni-M2) de l'exemple 5 sont réduits en présence d'hydrogène à 500° C pendant 2 heures. Ils sont ensuite placés à température ambiante dans un récipient clos balayé par de l'hydrogène puis mis au contact de 250 cm³ d'une solution de n-heptane dans lequel on a dissous 2 grammes d'étain sous forme de tétraméthylétain $(CH_3)_4$ Sn. L'ensemble est porté à ébullition et laissé à reflux pendant 2 heures.

Le solide est ensuite filtré, lavé 2 fois avec du n-heptane à température ambiante pendant 20 minutes, puis est séché à 150° C pendant 5 heures dans une étuve.

Le catalyseur F ainsi obtenu contient 0,43% en poids d'étain, ce qui correspond à un taux de fixation de l'étain de 21,5%.

*Exemple 11:*

Préparaton du catalyseur G (Sn-Ni-M3)

50 grammes du catalyseur B de l'exemple 6 sont soumis à un traitement identique à celui décrit dans l'exemple 10 précédent.

Le catalyseur G ainsi obtenu contient 0,39% en poids d'étain, ce qui correspond à un taux de fixation de l'étain de 19,5%.

*Exemple 12:*

Préparation du catalyseur H (Sn-Ni-M4)

50 grammes du catalyseur C de l'exemple 7 sont soumis à un traitement identique à celui décrit dans l'exemple 10 précédent.

Le catalyseur H ainsi obtenu contient 0,33% en poids d'étain, ce qui correspond à un taux de fixation de l'étain de 16,5%.

*Exemple 13:*

Préparation du catalyseur I (Sn-Ni-M5)

50 grammes du catalyseur D de l'exemple 8 sont soumis à un traitement identique à celui décrit dans l'exemple 10 précédent.

Le catalyseur I ainsi obtenu contient 0,28% en poids d'étain, ce qui correspond à un taux de fixation de l'étain de 14%.

*Exemple 14:*

Préparation du catalyseur J (Pb-Ni-M4) 50 grammes du catalyseur C de l'exemple 7 sont réduits en présence d'hydrogène à 500° C pendant 2 heures. Ils sont ensuite placés à température ambiante dans un récipient clos balayé par de l'hydrogène puis mis au contact de 250 cm³ d'une solution de n-heptane dans lequel on a dissous 5 grammes de plomb sous forme de tétraéthyl plomb $(C_2H_5)_4Pb$. L'ensemble est porté à ébullition et laissé à reflux pendant 2 heures.

Le solide est ensuite filtré, lavé 2 fois avec du n-heptane à température ambiante pendant 20 minutes, puis est séché à 150° C pendant 5 heures dans une étuve.

Le catalyseur J ainsi obtenu contient 1,55% en poids de plomb, ce qui correspond à un taux de fixation du plomb de 31%.

*Exemple 15:*

Préparation du catalyseur K (Sn-Pd-M4)

50 grammes du catalyseur E de l'exemple 9 sont soumis à un traitement comparable à celui décrit dans l'exemple 10 précédent, excepté en ce qui concerne la température de réduction qui est limitée à 450° C et la quantité d'étain présente au départ dans le n-heptane qui n'est que de 0,275 g.

Le catalyseur K ainsi obtenu contient 0,52% en poids d'étain, ce qui correspond à un taux de fixation de l'étain de 95,0%.

*Exemple 16:*

Préparation des catalyseurs L, N, O, P (Sn-$M_x$)

On dépose de l'étain sur les mordénites acides $M_2$ à $M_5$ préparées suivant les méthodes décrites respectivement dans les exemples 1 à 4, préalablement calcinées à 500° C sous air sec. Les mordénites $M_2$ à $M_5$ sont imprégnées «à sec», sans excès de solution, par une solution de tétraméthylétain $(CH_3)_4$ Sn dans le n-heptane.

La concentration de la solution de tétraméthylétain dans le n-heptane est choisie de manière à fixer les quantités d'étain mentionnées ci-après dans le tableau 1 pour chaque catalyseur à base des mordénites $M_2$ à $M_5$.

Les solides Sn-$M_2$, Sn-$M_3$, Sn-$M_4$, Sn-$M_5$ obtenus après imprégnation sont séchés à 150°C pendant 5 heures dans une étuve. Les caractéristiques des catalyseurs L, N, O, P ainsi obtenus figurent dans le tableau 1.

Tous les catalyseurs des exemples 1 à 16 dont les caractéristiques sont résumées dans le tableau 1 ont été testés et comparés dans un test de dismutation du toluène.

*Tableau 1*

| Catalyseur | Formule | $SiO_2/Al_2O_3$ | % poids Na | % poids Ni | % poids Pd | % poids Sn | % poids Pb |
|---|---|---|---|---|---|---|---|
| $A_o$ | $M_2$ | 10,6 | 0,22 | — | — | — | — |
| $B_o$ | $M_3$ | 14,6 | 0,20 | — | — | — | — |
| $C_o$ | $M_4$ | 25,2 | 0,11 | — | — | — | — |
| $D_o$ | $M_5$ | 58,6 | 0,009 | — | — | — | — |
| A | Ni-M2 | 10,6 | 0,22 | 1,81 | — | — | — |
| B | Ni-M3 | 14,6 | 0,20 | 2,11 | — | — | — |
| C | Ni-M4 | 25,2 | 0,11 | 1,10 | — | — | — |
| D | Ni-M5 | 58,6 | 0,09 | 0,43 | — | — | — |
| E | Pd-M4 | 25,2 | 0,11 | — | 0,10 | — | — |
| F | Sn-Ni-M2 | 10,6 | 0,22 | 1,81 | — | 0,43 | — |
| G | Sn-Ni-M3 | 14,6 | 0,20 | 2,11 | — | 0,39 | — |
| H | Sn-Ni-M4 | 25,2 | 0,11 | 1,10 | — | 0,33 | — |
| I | Sn-Ni-M5 | 58,6 | 0,09 | 0,43 | — | 0,28 | — |
| J | Pb-Ni-M4 | 25,2 | 0,11 | 1,10 | — | — | 1,55 |
| K | Sn-Pd-M4 | 25,2 | 0,11 | — | 0,10 | 0,52 | — |
| L | Sn-$M_2$ | 10,6 | 0,22 | — | — | 0,42 | — |
| N | Sn-$M_3$ | 14,6 | 0,20 | — | — | 0,38 | — |
| O | Sn-$M_4$ | 25,2 | 0,11 | — | — | 0,33 | — |
| P | Sn-$M_5$ | 58,6 | 0,09 | — | — | 0,28 | — |

*Exemple 17:*

Description du test catalytique de dismutation du toluène en phase vapeur.

Les tests sont effectués dans les conditions suivantes:
Pression totale: 20 bar (2 MPa)
Rapport molaire: $H_2$/toluène = 4
1re série de conditions pour comparer l'activité des catalyseurs à faible conversion
PPH = débit pondéral de toluène par gramme de catalyseur et par heure: 5 $h^{-1}$
Température variable, choisie pour obtenir une conversion globale inférieure à 15%.
2e série de conditions pour comparer la sélectivité des catalyseurs à forte conversion
PPH = 1,5 $h^{-1}$
Température variable choisie pour obtenir une conversion globale comprise entre 40 et 50%.

Un test de vieillissement accéléré du catalyseur est réalisé après détermination de son activité et de sa sélectivité, en portant la température à 520°C pendant 6 heures à PPH = 5 $h^{-1}$. L'activité du catalyseur «vieilli», déterminée ensuite dans les conditions déjà décrites ci-dessus, permet d'évaluer la stabilité dans le temps du catalyseur.

L'activité du catalyseur est jugée d'après la température nécessaire pour atteindre une conversion égale à 10% extrapolée à partir de divers points expérimentaux obtenus dans la première série de conditions et celle nécessaire pour obtenir une conversion égale à 45% dans la seconde série de conditions.

La sélectivité du catalyseur est jugée d'après la teneur totale en hydrocarbures saturés comportant de 1 à 9 atomes de carbone (pratiquement la plupart de ces composés comportent entre 1 et 7 atomes de carbone) obtenue à conversion élevée (45%). Plus cette teneur est élevée, plus la sélectivité est faible. La sélectivité peut également être appréciée par les valeurs des pourcentages des divers hydrocarbures saturés légers obtenus dans la coupe $C_1$-$C_4$.

La stabilité du catalyseur est jugée d'après l'augmentation de température nécessaire pour retrouver, après le vieillissement accéléré du catalyseur, les conversions atteintes avant ce vieillissement dans les deux séries de conditions décrites précédemment (correspondant respectivement à 10 et 45% de conversion totale).

*Exemple 18:*

Comparaison des catalyseurs A à D de référence.

Les activités à faible conversion (10%) et à forte conversion (45%) des catalyseurs A à D sont comparées dans un diagramme température en fonction du rapport $SiO_2/Al_2O_3$ (T(°C) = f ($SiO_2/Al_2O_3$)) présenté dans la figure 1.

Les sélectivités à forte conversion (45%) des catalyseurs A à D sont portées dans un diagramme pourcentage hydrocarbures saturés formés en

fonction du rapport $SiO_2/Al_2O_3$ (% $(HC_s)$ = f $(SiO_2/Al_2O_3)$) présenté dans la figure 2. Le tableau 2 qui présente les répartitions des gaz de $C_1$ à $C_4$ fournit également des informations sur la sélectivité.

La stabilité du catalyseur peut être appréciée dans la figure 1 en comparant les températures auxquelles sont obtenues les conversions de 10% et 45% avant le vieillissement du catalyseur à celles correspondantes obtenues après le vieillissement.

Il apparaît ainsi les principaux points suivants:

– L'activité et la sélectivité s'améliorent lorsque le rapport $SiO_2/Al_2O_3$ augmente de 10 à une valeur située entre 20 et 35 environ, puis se dégradent légèrement pour des valeurs supérieures à cette dernière, et retrouvent progressivement pour une valeur du rapport d'environ 50 un niveau voisin de celui obtenu pour une valeur du rapport de 10.

– Le tableau 2 indique que les hydrocarbures légers de $C_1$ à $C_4$ formés contiennent une forte proportion de $C_1 + C_2$, surtout après vieillissement du catalyseur. Cela peut vraisemblablement être attribué à une réaction parasite d'hydrogénolyse.

– La stabilité des divers catalyseurs dépend du niveau de conversion. Les catalyseurs A et B de rapports $SiO_2/Al_2O_3$ faibles (inférieurs à 15) ont une stabilité excellente à faible conversion mais nettement moins bonne à conversion élevée. En revanche, les catalyseurs dont le rapport $SiO_2/Al_2O_3$ est supérieur à environ 20 ont la même stabilité quelle que soit la conversion: la désactivation observée après vieillissement correspond à une augmentation de température de 50-60° C environ.

### Tableau 2

Pourcentages (en poids) produits et répartition des hydrocarbures saturés légers de $C_1$ à $C_4$ ($C_4^-$) à conversion élevée (45%) pour les catalyseurs A à D avant (av) et après (ap) vieillissement.

| Catalyseurs | | % $C_4^-$ * | % $C_1 + C_2$ dans $C_4^-$ | % $C_3$ dans $C_4^-$ | % $C_4$ dans $C_4^-$ |
|---|---|---|---|---|---|
| A | av | 12,0 | 30,5 | 37,6 | 32,9 |
| | ap | 3,1 | 68,2 | 23,6 | 8,2 |
| B | av | 4,3 | 31,2 | 46,9 | 21,9 |
| | ap | 8,5 | 81,5 | 14,5 | 4,0 |
| C | av | 1,3 | 19,2 | 48,4 | 32,4 |
| | ap | 5,8 | 67,9 | 26,8 | 5,3 |
| D | av | 3,9 | 39,6 | 33,1 | 27,3 |
| | ap | 14,6 | 89,1 | 7,6 | 3,3 |

* % $C_4^-$ en poids dans les produits formés.

### Exemple 19:

Comparaison des catalyseurs F à I contenant de l'étain et du nickel et des catalyseurs de référence A à D.

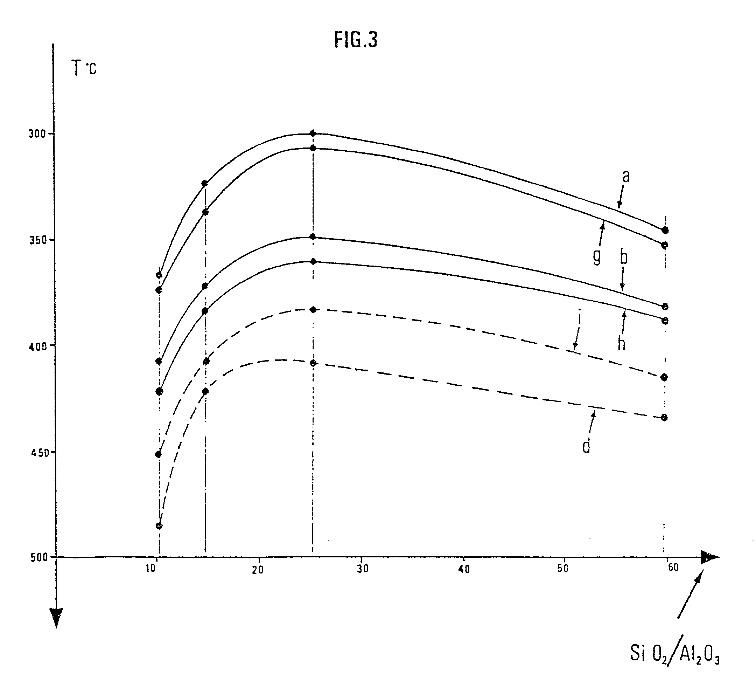

La figure 3 montre que l'introduction d'étain dans les mordénites acides au nickel caractérisées par des rapports molaires $SiO_2/Al_2O_3$ différents allant de 10,6 à 58,6 se traduit dans tous les cas:

– par une légère diminution de l'activité du catalyseur qui correspond approximativement à la nécessité d'augmenter la température de 10° C;

– par une nette amélioration de la stabilité après le vieillissement forcé du catalyseur: la perte d'activité du catalyseur après le vieillissement correspond à une augmentation de température de 50-60° C dans le cas des catalyseurs de référence A à D (Ni-mordénite) contre 20 à 30° C seulement avec les catalyseurs F à I (Sn-Ni-mordénite).

Les tableaux 3 et 4 indiquent les rendements en produits saturés totaux de $C_1$ à $C_9$ formés, en produits saturés légers de $C_1$ à $C_4$ formés et la répartition de chacun des hydrocarbures de $C_1$ à $C_4$ dans la fraction $C_4^-$ ($C_1$ à $C_4$). Ils montrent que la présence d'étain permet d'améliorer considérablement la sélectivité des mordénites au nickel en diminuant fortement la production de composés non aromatiques.

Le tableau 4 indique que la proportion de $C_1 + C_2$ formée dans les gaz légers en $C_4^-$ diminue fortement avec les catalyseurs F à I par rapport aux catalyseurs A à D (voir tableau 2), ce qui démontre que l'étain modifie les propriétés du nickel.

*(Tableaux en page suivante)*

### Exemple 20:

Comparaison des catalyseurs E (Pd-mordénite) et K (Sn-Pd-mordénite).

Le tableau 5 ci-après présente les principales caractéristiques d'activité, sélectivité et stabilité des deux catalyseurs avant (av) et après (ap) vieillissement.

Les pourcentages des produits sont exprimés en poids et la répartition des hydrocarbures légers de $C_1$ à $C_4$ ($C_4^-$) est en pourcent en poids dans la fraction $C_4^-$.

Les résultats montrent que le catalyseur à base de palladium et d'étain, selon la présente invention, possède une activité initiale voisine (quoique légèrement inférieure) de celle du catalyseur de référence; mais sa sélectivité et sa stabilité dans le temps sont très nettement améliorées.

### Exemple 21:

Comparaison des catalyseurs C (Ni-mordénite) et J (Pb-Ni-mordénite).

Le tableau 6 ci-après présente les principales caractéristiques d'activité, sélectivité et stabilité des deux catalyseurs avant (av) et après (ap) vieillissement.

Les pourcentages des produits sont exprimés en poids et la répartition des hydrocarbures légers de $C_1$ à $C_4$ ($C_4^-$) est en pourcent en poids dans la fraction $C_4^-$.

Les résultats montrent que le catalyseur à base de nickel et plomb, selon la présente invention, possède une activité initiale légèrement inférieure à celle du catalyseur de référence, mais possède en revanche une sélectivité et une stabilité dans le temps nettement améliorées.

*Tableau 3*

Pourcentages (en poids) produits d'hydrocarbures saturés de $C_1$ à $C_9$ dans le cas des catalyseurs F à I comparés à ceux des catalyseurs de référence A à D avant vieillissement (avant) et après vieillissement (après) pour une conversion de 45%.

| Catalyseurs | | % de $C_1$ à $C_9$ saturés formés | |
|---|---|---|---|
| $SiO_2/Al_2O_3$ | | avant | après |
| 10,6 | A ($Ni-M_2$)<br>F ($Sn-Ni-M_2$) | 17,1<br>1,9 | 14,0<br>1,3 |
| 14,6 | B ($Ni-M_3$)<br>G ($Sn-Ni-M_3$) | 5,1<br>1,2 | 9,5<br>0,7 |
| 25,2 | C ($Ni-M_4$)<br>H ($Sn-Ni-M_4$) | 3,0<br>0,5 | 7,0<br>0,4 |
| 58,6 | D ($Ni-M_5$)<br>I ($Sn-Ni-M_5$) | 5,9<br>0,9 | 15,6<br>0,7 |

*Tableau 4*

Pourcentages (en poids) produits et répartition des hydrocarbures saturés légers de $C_1$ à $C_4$ ($C_4^-$) à conversion élevée (45%) pour les catalyseurs F à I avant (av) et après (ap) vieillissement.

| Catalyseurs | | % $C_4^-$ | % $C_1+C_2$ dans $C_4^-$ | % $C_3$ dans $C_4^-$ | % $C_4$ dans $C_4^-$ |
|---|---|---|---|---|---|
| F | av | 1,5 | 25,3 | 50,6 | 24,1 |
| | ap | 1,1 | 22,6 | 52,1 | 25,3 |
| G | av | 1,0 | 16,7 | 59,3 | 24,0 |
| | ap | 0,6 | 10,3 | 68,2 | 21,5 |
| H | av | 0,4 | 6,1 | 76,3 | 17,6 |
| | ap | 0,3 | 5,2 | 77,8 | 17,0 |
| I | av | 0,7 | 12,4 | 69,1 | 18,5 |
| | ap | 0,5 | 9,5 | 72,4 | 18,1 |

*Tableau 5*

| Catalyseurs | | T°C 10% conversion | T°C 45% conversion | % $C_1$ à $C_9$ (*) | % $C_4^-$ (*) | % $C_1+C_2$ dans $C_4^-$ (*) | % $C_3$ dans $C_4^-$ (*) | % $C_4$ dans $C_4^-$ (*) |
|---|---|---|---|---|---|---|---|---|
| E ($Pd-M_4$) | av | 295 | 345 | 6,5 | 5,6 | 6,1 | 73,8 | 20,1 |
| | ap | 350 | 400 | 5,8 | 5,1 | 5,5 | 73,6 | 20,9 |
| K ($Sn-Pd-M_4$) | av | 300 | 350 | 0,8 | 0,7 | 5,1 | 74,4 | 20,5 |
| | ap | 315 | 370 | 0,7 | 0,6 | 4,5 | 75,1 | 20,4 |

(*) valeurs correspondant à 45% de conversion.

Tableau 6

| Catalyseurs | | T°C 10% conversion | T°C 45% conversion | % $C_1$ à $C_9$ (*) | % $C_4^-$ (*) | % $C_1+C_2$ dans $C_4^-$ (*) | % $C_3$ dans $C_4^-$ (*) | % $C_4$ dans $C_4^-$ (*) |
|---|---|---|---|---|---|---|---|---|
| C ($Ni-M_4$) | av | 300 | 350 | 3,0 | 1,3 | 19,2 | 48,4 | 32,4 |
| | ap | 350 | 410 | 7,0 | 5,8 | 67,9 | 26,8 | 5,3 |
| J ($Pb-Ni-M_4$) | av | 315 | 360 | 0,8 | 0,6 | 9,6 | 66,0 | 24,4 |
| | ap | 340 | 390 | 0,7 | 0,5 | 10,9 | 73,9 | 15,2 |

(*) valeurs correspondant à 45% de conversion.

*Exemple 22:*

Comparaison des catalyseurs $A_o$ à $D_o$ de référence.

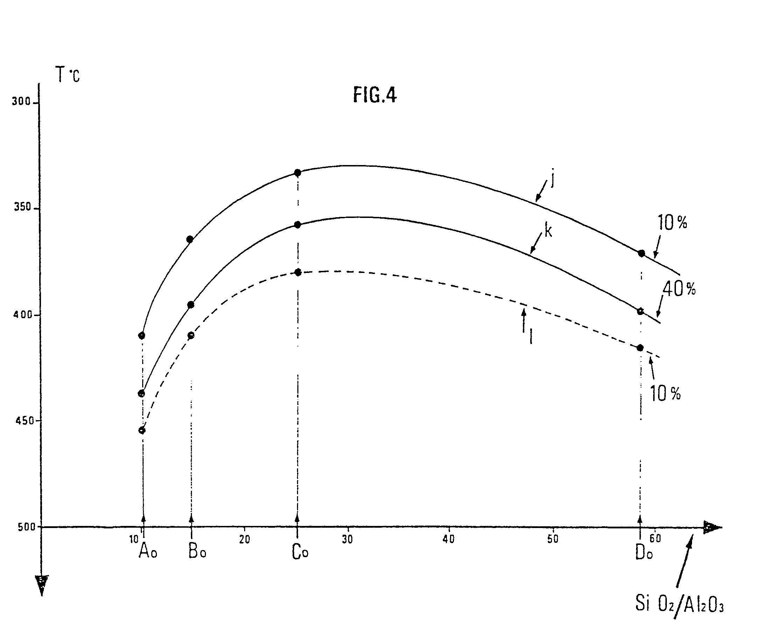

Les activités à faible conversion (10%) et à forte conversion (40%) des catalyseurs $A_o$ à $D_o$ sont comparées dans un diagramme température en fonction du rapport $SiO_2/Al_2O_3$ ($T(°C) = f(SiO_2/Al_2O_3)$) présenté sur la figure 4.

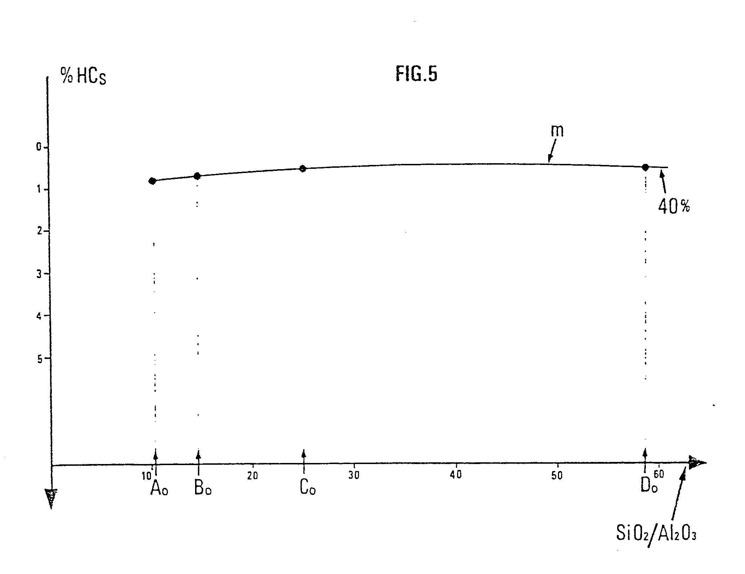

Les sélectivités à forte conversion (40%) des catalyseurs $A_o$ à $D_o$ sont portées dans un diagramme pourcentage d'hydrocarbures saturés formés en fonction du rapport $SiO_2/Al_2O_3$ ($\%(HC_s) = f(SiO_2/Al_2O_3)$) présenté sur la figure 5.

Le tableau 7 qui présente les répartitions des gaz de $C_1$ à $C_4$ fournit également des informations sur la sélectivité.

La stabilité du catalyseur peut être appréciée dans la figure 4 en comparant les températures auxquelles est obtenue la conversion de 10% avant le vieillissement du catalyseur (courbe j) à celles correspondantes obtenues après le vieillissement (courbe I).

La courbe relative à une conversion de 40% après vieillissement n'a pas été portée sur la figure 4 par suite de la difficulté d'atteindre cette conversion avec certains catalyseurs, notamment pour le catalyseur $A_o$ ayant un rapport $SiO_2/Al_2O_3$ de 10,6, les catalyseurs perdant très vite leur activité au cours du temps au-delà de 440-450° C.

Il apparaît ainsi les principaux points suivants:

— l'activité des catalyseurs $A_o$ à $D_o$ s'améliore lorsque le rapport $SiO_2/Al_2O_3$ augmente de 10 à une valeur située entre 20 et 35 environ, puis diminue pour des valeurs supérieures à 35 environ,

— la sélectivité est bonne pour tous les catalyseurs $A_o$ à $D_o$: moins de 1% poids de produits saturés sont formés dans tous les cas,

— les hydrocarbures légers de $C_1$ à $C_4$ formés contiennent une faible proportion de $C_1+C_2$. Celle-ci tend cependant à être d'autant plus élevée que la température est plus haute.

Tableau 7

Pourcentages (en poids) produits et répartition des hydrocarbures saturés légers de $C_1$ à $C_4$ à conversion élevée (40%) pour les catalyseurs $A_o$ à $D_o$ avant vieillissement.

| Catalyseurs | % $C_4^-$ * | % $C_1+C_2$ dans $C_4^-$ | % $C_3$ dans $C_4^-$ | % $C_4$ dans $C_4^-$ |
|---|---|---|---|---|
| $A_o$ | 0,7 | 20,1 | 53,2 | 26,7 |
| $B_o$ | 0,6 | 14,2 | 60,7 | 25,1 |
| $C_o$ | 0,5 | 7,3 | 75,6 | 17,1 |
| $D_o$ | 0,5 | 12,8 | 68,6 | 18,6 |

* % $C_4^-$ en poids dans les produits formés.

*Exemple 23:*

Comparaison des catalyseurs L, N, O, P contenant de l'étain aux catalyseurs $A_o$ à $D_o$ et A à D et F à I.

Les activités à faible conversion (10%) et à forte conversion (40%) des catalyseurs L, N, O et P sont très légèrement inférieures à celles des catalyseurs de référence correspondants $A_o$ à $D_o$ ayant le même rapport $SiO_2/Al_2O_3$. Ces activités légèrement moins bonnes se traduisent par la nécessité d'augmenter les températures d'environ 5° C, par rapport à celles des catalyseurs $A_o$ à $D_o$, pour obtenir les niveaux de conversions souhaités (10% ou 40%).

Comme pour les catalyseurs de référence $A_o$ à $D_o$, la stabilité des catalyseurs L, N, O et P n'a pas pu être évaluée à forte conversion (40%), ces catalyseurs L, N, O et P perdant trop rapidement leur activité à conversion élevée, donc à température élevée.

La stabilité des catalyseurs L, N, O et P à 10% de conversion est identique à celle des catalyseurs correspondants $A_o$, $B_o$, $C_o$ et $D_o$.

La sélectivité à forte conversion (40%) des catalyseurs L, N, O et P avant vieillissement est très voisine de celle des catalyseurs de référence $A_o$ à $D_o$ correspondants: la production d'hydrocarbures saturés est à 0,1% près la même que celle des catalyseurs $A_o$ à $D_o$, la proportion de $C_1 + C_2$ formée dans les gaz légers $C_4^-$ est dans tous les cas légèrement plus faible d'une quantité voisine de 2% environ.

La comparaison des catalyseurs F à I selon la présente invention montre que la plus grande partie de l'activité et de la stabilité des catalyseurs A à D est conservée et que la sélectivité est très nettement améliorée vis-à-vis des catalyseurs A à D.

Les catalyseurs F à I ont une activité et une stabilité nettement améliorées vis-à-vis des catalyseurs $A_o$ à $D_o$ et L, N, O et P respectivement. La sélectivité des catalyseurs F à I est très voisine de celle des catalyseurs $A_o$ à $D_o$ et L, N, O et P respectivement.

Les catalyseurs F à I sont donc globalement supérieurs vis-à-vis des catalyseurs de comparaison, si l'on se réfère aux trois qualités de base des catalyseurs: activité, stabilité et sélectivité.

## Revendications

1. Procédé de dismutation ou de transalkylation de composés alkylaromatiques, dans des conditions de dismutation ou de transalkylation, caractérisé en ce que l'on opère en présence d'un catalyseur renfermant

a) au moins une mordénite acide dont le rapport molaire global $SiO_2/Al_2O_3$ est de 9 à 80 et dont la teneur en sodium est inférieure à 1% en poids,

b) au moins un métal choisi dans le groupe formé par le nickel, le palladium et les métaux du groupe IB de la classification périodique des éléments,

c) au moins un métal choisi parmi les métaux du groupe IVA de la classification périodique des éléments.

2. Procédé selon la revendication 1, caractérisé en ce que le catalyseur est préparé en introduisant au moins un métal du groupe IVA de la classification périodique des éléments à l'aide de complexe organique de ce ou ces métaux.

3. Procédé selon l'une des revendications 1 ou 2 dans lequel la mordénite acide renferme moins de 0,5% en poids de sodium.

4. Procédé selon l'une des revendications 1 à 3 dans lequel la mordénite acide a un rapport molaire global $SiO_2/Al_2O_3$ de 12 à 60.

5. Procédé selon l'une des revendications 1 à 4 dans lequel le catalyseur renferme:

a) de 30 à 99,9% en poids de mordénite acide,

b) au moins un métal choisi dans le groupe formé par le nickel, le palladium et l'argent,

c) au moins un métal du groupe IVA de la classification périodique des éléments choisi parmi l'étain, le plomb et le germanium.

6. Procédé selon l'une des revendications 1 à 5 dans lequel le catalyseur renferme un liant choisi dans le groupe formé par les argiles, les alumines, la silice et les silices-alumines.

7. Procédé selon la revendication 6 dans lequel le catalyseur renferme de 3 à 50% en poids de liant.

8. Procédé selon l'une des revendications 1 à 7 dans lequel le catalyseur renferme en poids:

a) de 0,005 à 25% d'au moins un métal choisi dans le groupe formé par le nickel, le palladium et les métaux du groupe IB de la classification périodique des éléments,

b) de 0,05 à 10% d'au moins un métal du groupe IVA de la classification périodique des éléments.

9. Procédé selon l'une des revendications 1 à 8 appliqué à la dismutation du toluène.

10. Procédé selon l'une des revendications 1 à 8 appliqué à la transalkylation du toluène et des hydrocarbures alkylaromatiques ayant au moins 9 atomes de carbone par molécule.

## Patentansprüche

1. Verfahren zur Disproportionierung oder Transalkylierung von alkylaromatischen Verbindungen unter Disproportionierungs- oder Transalkylierungsbedingungen, dadurch gekennzeichnet, dass man in Gegenwart eines Katalysators arbeitet, der enthält:

a) mindestens einen sauren Mordenit, dessen molares Gesamtverhältnis $SiO_2/Al_2O_3$ zwischen 9 und 80 und dessen Natriumgehalt unterhalb 1 Gew.-% liegt,

b) zumindest ein Metall aus der Gruppe Nickel, Palladium und Metallen der Gruppe IB des periodischen Systems der Elemente,

c) zumindest ein Metall aus den Metallen der Gruppe IVA des periodischen Systems der Elemente (gemäss «Handbook of Chemistry and Physics» 37. Aufl. 1955-56, S. 392-393).

2. Verfahren nach Anspruch 1, dadurch gekennzeichnet, dass der Katalysator hergestellt wird, indem man zumindest ein Metall der Gruppe IVA des periodischen Systems der Elemente mit Hilfe eines organischen Komplexes dieses Metalls oder dieser Metalle einführt.

3. Verfahren nach einem der Ansprüche 1 oder 2, dadurch gekennzeichnet, dass der saure Mordenit weniger als 0,5 Gew.-% Natrium enthält.

4. Verfahren nach einem der Ansprüche 1 bis 3, dadurch gekennzeichnet, dass der saure Mordenit ein molares Gesamtverhältnis $SiO_2/Al_2O_3$ von 12 bis 60 hat.

5. Verfahren nach einem der Ansprüche 1 bis 4, dadurch gekennzeichnet, dass der Katalysator enthält:

a) 30 bis 99,9 Gew.-% sauren Mordenit,

b) zumindest ein Metall aus der Gruppe Nickel, Palladium und Silber,

c) zumindest ein Metall der Gruppe IVA des periodischen Systems der Elemente aus der Gruppe Zinn, Blei und Germanium.

6. Verfahren nach einem der Ansprüche 1 bis 5, dadurch gekennzeichnet, dass der Katalysator ein Bindemittel aus der Gruppe Tone, Aluminiumoxide, Siliciumdioxid und Siliciumdioxid-Aluminiumoxide enthält.

7. Verfahren nach Anspruch 6, dadurch gekennzeichnet, dass der Katalysator 3 bis 50 Gew.-% Bindemittel enthält.

8. Verfahren nach einem der Ansprüche 1 bis 7, dadurch gekennzeichnet, dass der Katalysator in Gewichtsanteilen enthält:

a) 0,005 bis 25% von zumindest einem Metall aus der Gruppe Nickel, Palladium und den Metallen der Gruppe IB des periodischen Systems der Elemente,

b) 0,05 bis 10% zumindest eines Metalls der Gruppe IVA des periodischen Systems der Elemente.

9. Verfahren nach einem der Ansprüche 1 bis 8, angewandt auf die Disproportionierung von Toluol.

10. Verfahren nach einem der Ansprüche 1 bis 8, angewandt auf die Transalkylierung von Toluol und von alkylaromatischen Kohlenwasserstoffen mit mindestens 9 Kohlenstoffatomen pro Molekül.

## Claims

1. A process for dismutation or transalkylation of alkylaromatic compounds, in dismutation or transalkylation conditions, characterized in that it is conducted in the presence of a catalyst containing:

a) at least one acid mordenite whose total $SiO_2/Al_2O_3$ molar ratio is from 9 to 80 and whose sodium content is lower than 1% by weight,

b) at least one metal selected from the group formed of nickel, palladium and metals of group IB of the periodic classification of elements,

c) at least one metal selected from the metals of group IVA of the periodic classification of elements.

2. A process according to claim 1, characterized in that the catalyst is prepared by introducing at least one metal from group IVA of the periodic classification of elements by means of an organic complex of this or these metals.

3. A process according to one of claims 1 or 2 wherein the acid mordenite contains less than 0.5% by weight of sodium.

4. A process according to one of claims 1 to 3 wherein the acid mordenite has a total $SiO_2/Al_2O_3$ molar ratio of 12 to 60.

5. A process according to one of claims 1 to 4 wherein the catalyst contains:

a) from 30 to 99.9% by weight of acid mordenite,

b) at least one metal selected from the group formed of nickel, palladium and silver,

c) at least one metal from group IVA of the periodic classification of elements, selected from tin, lead and germanium.

6. A process according to one of claims 1 to 5 wherein the catalyst contains a binding agent selected from the group formed of clays, aluminas, silica and silica-aluminas.

7. A process according to claim 6 wherein the catalyst contains 3 to 50% by weight of binding agent.

8. A process according to one of claims 1 to 7 wherein the catalyst contains by weight:

a) from 0.005 to 25% of at least one metal selected from the group formed of nickel, palladium and the metals of group IB of the periodic classification of elements,

b) from 0.05 to 10% of at least one metal from group IVA of the periodic classification of elements.

9. A process according to one of claims 1 to 8 applied to toluene dismutation.

10. A process according to one of claims 1 to 8 applied to transalkylation of toluene and alkylaromatic hydrocarbons having at least 9 carbon atoms per molecule.

## FIG.1

FIG.2

## FIG.3

FIG.4

FIG.5